## Europäisches Patentamt
## European Patent Office
## Office européen des brevets

(19)

(11) Veröffentlichungsnummer : **0 109 352**
**B1**

(12) # EUROPÄISCHE PATENTSCHRIFT

(45) Veröffentlichungstag der Patentschrift :
11.11.87

(21) Anmeldenummer : 83810219.2

(22) Anmeldetag : 25.05.83

(51) Int. Cl.⁴ : **A 61 L 2/20**// A01 N37/16

(54) Verfahren zum Sterilisieren von Utensilien, insbesondere aus thermolabilen Materialien.

(30) Priorität : 16.11.82 CH 6673/82

(43) Veröffentlichungstag der Anmeldung :
23.05.84 Patentblatt 84/21

(45) Bekanntmachung des Hinweises auf die Patenterteilung : 11.11.87 Patentblatt 87/46

(84) Benannte Vertragsstaaten :
AT BE CH DE FR GB IT LI LU NL SE

(56) Entgegenhaltungen :
DIE PHARMAZIE, Nr. 31, Heft 7, 1976, Seiten 491-492,
VEB Verlag Volk und Gesundheit, Berlin, DE;
M.SPRÖSSIG et al.: "Untersuchungen über die sterilisierende Wirkung von gasförmiger Peressigsäure an
papierverpacktem Material"
DIE PHARMAZIE, Nr. 29, Heft 2, 1974, Seiten 132-137,
VEB Verlag, Berlin, DE; M.SPRÖSSIG et al.: "Versuche und Betrachtungen zur Sterilisation thermolabiler
Gegenstände mit Peressigsäure"
H.P. WEUFFEN: "Handbuch der Desinfektion und
Sterilisation", Band 1, 1972, Seiten 173-175, VEB
Verlag Volk und Gesundheit, Berlin, DE
"Handbuch der Desinfektion und Sterilisation"
K.H. WALLHÄUSSER: "Sterilisation, Desinfektion,
Konservierung", 2. Auflage, 1978, Seite 404, Georg
Thieme Verlag, Stuttgart, DE.

(73) Patentinhaber : Kantonsspital St. Gallen
Rorschacher Strasse
CH-9007 St. Gallen (CH)

(72) Erfinder : Mouron, Rudolf
Tuchgasse 4
CH-9220 Bischofszell (CH)
Erfinder : Huwiler, Beat
Höhenstrasse 5
CH-9320 Arbon (CH)
Erfinder : Sonnabend, Wolfgang
Bruggwaldpark 44
CH-9008 St. Gallen (CH)

(74) Vertreter : White, William et al
PATENTANWALTS-BUREAU ISLER AG Postfach 6940
Walchestrasse 23
CH-8023 Zürich (CH)

## Beschreibung

Die vorliegende Erfindung betrifft ein Verfahren zum Sterilisieren von Utensilien, die aus thermolabilem Material bestehen oder Teile aus solchem Material besitzen.

In der Medizin werden Instrumente, Ausrüstungen und Prothesen sterilisiert und in sterilem Zustand aufbewahrt, bis sie ein nächstes Mal verwendet werden. Diese Utensilien werden, sofern sie aus hitzebeständigem Material hergestellt sind, durch feuchte oder trockene Hitze sterilisiert. Viele andere Utensilien sind jedoch nicht hitzebeständig (Gummi, Polyäthylen, PVC oder dergl.) und müssen auf chemisch-physikalische Art, d. h. durch sogenannte Kaltsterilisation sterilisiert werden. Hierzu werden bisher Formaldehyd oder Aethylenoxyd als mikrobizide Mittel eingesetzt. Diese Stoffe sind jedoch hoch toxisch und deren mögliche Cancerogenität wird heute intensiv diskutiert. Neben Formaldehyd und Aethylenoxyd wurde auch Peressigsäure verwendet, konnte sich aber in der Humanmedizin bis heute nicht durchsetzen, einerseits wohl wegen der Explosionsgefahr konzentrierter Peressigsäure, andererseits wegen der relativ raschen Zersetzung verdünnter Peressigsäure, bzw. deren Korrosität.

In Pharmazie 31, Heft 7, Seiten 401f wird Peressigsäure als Sterilisationsmittel angegeben und zwar soll demnach gasförmige Peressigsäure in einem geschlossenen Behälter verwendet werden. Dazu wurde WOLFASTERIL, also eine 40 %ige Peressigsäure in einer Schale zur Verdampfung gebracht. Versuche wurden bei einem Anfangsvakuum von 15 mm Hg bei 22° oder 37° während 2 bis 0,75 h gemacht. Weiter wird aber angegeben, dass die Sterilisation in etwa 16 h bei Normaldruck und Raumtemperatur durchgeführt werden sollte.

Es ist bekannt, dass der Flammpunkt einer 40 %igen Lösung bei 40 °C liegt. Man muss daher aus den obigen Angaben schliessen, dass es sich höchstens um Laborversuche gehandelt haben kann, denn sonst wäre die erwähnung von 37 °C eine sehr gefährliche Angabe und ist keineswegs für die Praxis in Spitälern, Arztpraxen oder Fabriken geeignet.

Andere Hinweise auf Verwendung der Peressigsäure finden sich in « Sterilisation, Desinfektion, Konservierung », 1978, von K. H. Wallhäuser, Seite 404. Es wird die antimikrobielle Wirkung der Peressigsäure in kleinen Konzentrationen angesprochen (d. h. Peressigsäuregehalt von 1 mg/Liter Luft und mehr als 40 % relative Feuchte), wobei aber das Sterilgut nicht verpackt wurde. Hier handelt es sich also nicht um eine Sterilisation von verpacktem Gut im medizinischen Sinne.

Schliesslich findet sich im « Handbuch der Desinfektion und Sterilisation », 1972, von W. Weuffen, Seite 174f, ein weiterer Hinweis auf die Verwendung der Peressigsäure. Hier werden verschiedene Bereiche der Medizin genannt, in denen Peressigsäure verwendet werden kann. Zudem werden Grenzwerte der Konzentration, die in offenen Systemen verwendet werden, genannt und zwar nicht höher als 2 % Peressigsäure. Weuffen erläutert auch, dass das Abpumpen der Luft das Eindringen von Aerosolen und Dämpfen in poröse Oberflächen verbessert. Dabei ist aber nicht von verpacktem Sterilgut die Rede. Deshalb, und auch weil von offenen Systemen die Rede ist, handelt es sich hier auch nicht um ein Sterilisationsverfahen im medizinischen Sinne.

Trotz dieser mehrfachen Hinweise auf die Sterilisationseigenschaften der Peressigsäure ist bis heute kein Verfahren zur Sterilisation bekannt geworden, das sich gefahrlos industriell und in der Humanmedizin anwenden lässt.

Es ist demgemäss eine Aufgabe der Erfindung, die hervorragenden Eigenschaften der Peressigsäure bei mikrobizider Anwendung zu benützen, um eine zuverlässige und schnelle Sterilisation mit nur geringer Umweltbelastung zu ermöglichen. Dabei sollen Instrumente, Prothesen und Geräte der Humanmedizin in Sterilbeutel verpackt sterilisierbar sein, d. h. alle Mikroorganismen inklusive deren Dauerform sollen abgetötet werden können.

Erfindungsgemäss wird dies dadurch erreicht, dass das zu sterilisierende Gut zuerst in Sterilbeutel verpackt und dann in eine luftdicht abschliessbare Kammer eingebracht wird, dass darauf die Luft in der Kammer bis auf einen Druck im Bereich von 10-100 mbar evakuiert wird und wenigstens das Gut auf 40-42 Grad Celsius erwärmt wird, dass dann Peressigsäurelösung in einer Konzentration im Bereich von 4-7 % Peressigsäure und 93-96 % Wasser in der Kammer bei der genannten Temperatur zur Verdampfung gebracht wird, so dass bei einer Konzentration der verdampften Peressigsäure zwischen 0,1 bis 3,0 mg/Liter ein selbständiger Druckanstieg auf 50-140 mbar bei 40 °C bewirkt wird, und dass das verpackte Gut während einer Sterilisierzeit von höchstens 150 Minuten in dieser Umgebung belassen wird.

Eine Vorrichtung zur beispielsweisen Erläuterung der Erfindung wird nachfolgend anhand der Zeichnung erläutert. Es zeigen :

Fig. 1 eine schematische Darstellung einer Vorrichtung zur Durchführung des erfindungsgemässen Verfahrens,

Fig. 2 ein Zeitdiagramm für die verschiedenen Verfahrensschritte, und

Fig. 3 ein Druck-Zeit-Diagramm

Das hauptsächliche Problem, das zu lösen war, bestand darin, Peressigsäure unter Umgehung explosiver Zersetzung durch hohe Temperaturen in die Gasphase zu überführen, um damit keimdicht verpacke Gegenstände zu sterilisieren und dadurch den sterilen Zustand über längere Zeit aufrecht zu erhalten.

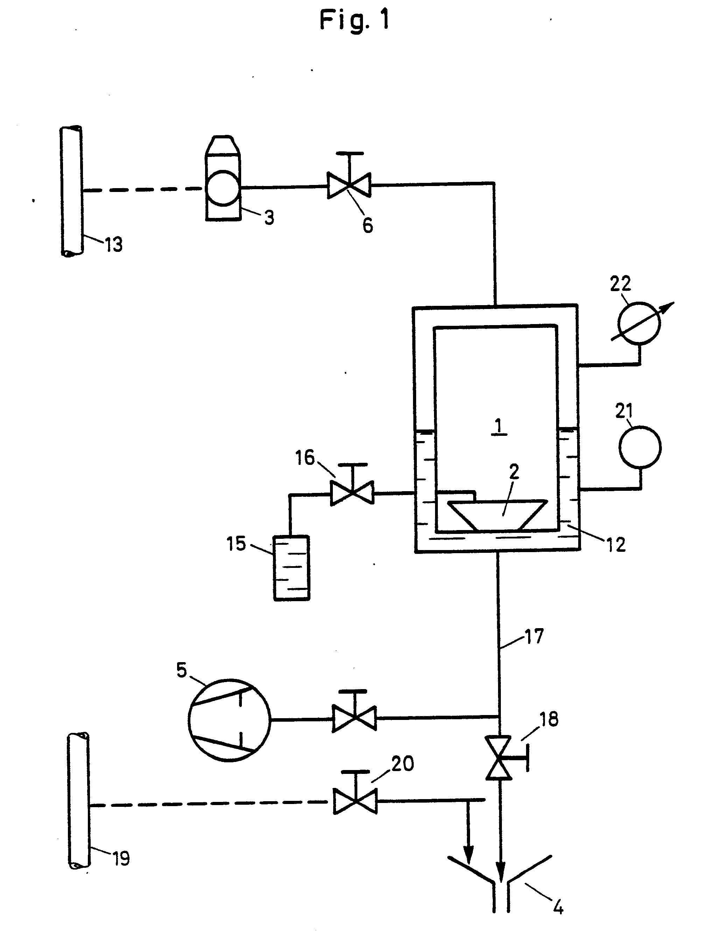

Die in Fig. 1 dargestellte Vorrichtung besteht aus einer luftdicht verschliessbaren Kammer 1 mit einer Doppelwand zur Bildung eines Wasserbades 12. In dieser Kammer 1 befindet sich ein schalenförmiger Behälter 2 für Peressigsäure. Von einem Drucklufnetz 13 wird eine Zuleitung gespeist, die über einen Druckregler 3 und ein Absperrventil 6 in die Kammer 1 geführt ist.

Aus einem Peressigsäure-Vorratsbehälter 15 wird Peressigsäure über ein Absperrventil 16 in den schalenförmigen Behälter 2 eingeleitet. Zur Evakuierung der Kammer 1 ist eine Ableitung 17 über ein weiteres Absperrventil 18 mit einer Wasserstrahlpumpe 4 verbunden, die aus einem Wassernetz 19 über ein Absperrventil 20 gespeist ist.

Mit einer Drehschiebervakuumpumpe 5 kann in der Kammer ein Vakuumendwert von etwa 1 mbar erzeugt werden.

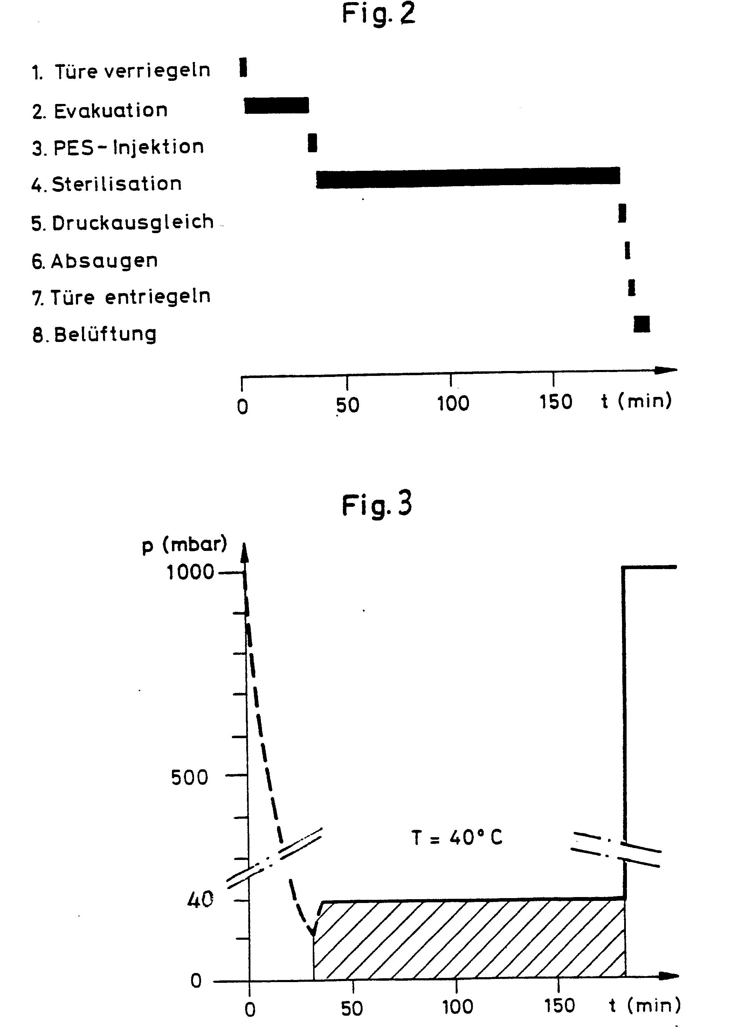

Anhand der Fig. 1-3 wird nachfolgend die Betriebsweise der Vorrichtung erläutert. Vorerst wird das zu sterilisierende Gut, das in Sterilbeuteln verpackt ist, in die Kammer 1 verbracht. Nach dem luftdichten Verschliessen der Kammer 1 wird mittels der Wasserstrahlpumpe 4 und der Drehschiebervakuumpumpe 5 ein Endvakuum von etwa 30 mbar erzeugt, das gemäss Fig. 2 nach etwa 15 Minuten erreicht wird. Gleichzeitig wird über das Wasserbad 12 Wärme in die Kammer eingeleitet, so dass nach Ablauf der genannten Zeit der gesamte Innenraum auf einer Temperatur von etwa 40 °C liegt.

Diese Aufwärmung verhindert ein Auskondensieren des Peressigsäuredampfes am Sterilgut, dazu öffnen sich die Poren der Sterilbeutel, damit die Restluft austreten kann und nicht einen Innendruck erzeugt, der zur Zerstörung der Sterilbeutel führen kann. Auch ist das Absaugen der Luft aus den Sterilbeuteln für die nachträgliche Sterilisation unerlässlich. Es hat sich gezeigt, dass die Zeit von 15 Minuten für diesen Zweck geeignet ist.

Nach Erreichen des Vakuums mit einem Druck von etwa 30 mbar wird Peressigsäure-Lösung von 4 % Peressigsäure und 96 % Wasser aus dem Behälter 15 über das Absperrventil 16 in den schalenförmigen Behälter 2 eingeleitet. Die Beschickung kann dabei derart bemessen werden, dass die zugeführte Menge genau zur Erzielung des Dampfdruckes ausreicht.

An die Einrichtung sind folgende Bedingungen zu stellen : Die Peressigsäure-Lösung muss langsam einströmen. Der Totraum der Rohre muss so klein wie möglich sein, um Restmengen zu vermeiden. Der schalenförmige Behälter 2 soll eine grosse Verdampferfläche bilden und leicht zu reinigen sein. Wenn die Einleitung der Peressigsäure-Lösung über einen Zerstäuber erfolgt, kann die Verdampfung beschleunigt werden.

Der stabile Zustand wird bei einer Lösung mit der oben genannten Konzentration bei etwa 75 mbar erreicht. Dieser Zustand muss nun während etwa 150 Minuten aufrechterhalten werden. Sollte ein Druckanstieg über dieser Grenze am Druckmesser 21 festgestellt werden, so würde dies ein Leck in der Kammer anzeigen. Die gleichmässige Temperatur von 40° C kann mittels eines Thermometers 22 festgestellt werden.

Nach Ablauf der Zeit von 150 Minuten wird die Peressigsäure-Lösung aus der Kammer 1 abgesaugt.

Vorteilhafterweise wird eine Luftwäsche in der Kammer durchgeführt, bevor die Türe geöffnet wird, um eine Geruchsbelästigung oder eine Reizung durch die Peressigsäure zu vermeiden, und schliesslich wird Luft aus dem Netz 13 zugeführt, um den Druckausgleich herzustellen.

Zum Nachweis der wirksamen Sterilisierung wurden insgesamt 598 Proben mit Peressigsäure-Lösung überprüft. Davon waren 540 in Folien aus « Lupolen » der Firma BASF, 41 in Sterilisierpapierbeuteln der Papierfabrik Cham AG und 17 in Steriking-Sterilisationsverpackungsmaterial der Firma Kaija Auterinen eingepackt. Als Keimträger dienten u. a. Kapillarröhrchen mit einem Innendurchmesser von 0,2 mm und einer Länge von 50 mm.

In einem ersten Versuch wurde eine Peressigsäure-Lösung bestehend aus 4 % Peressigsäure, 33 % Aethanol und 63 % Wasser verwendet. Das Endvakuum wurde bei 50 mbar erreicht, und der Dampfdruck erzeugte einen Druckanstieg von 50 mbar. Alle Keime, nämlich Pseudomonas aeruginosa, E. Coli, Klebsiella oxytoea, Serratia marcescens, Enterobacter cloacae, Staphilokokken aurens, Enterokokken und B. Stearothermophilus Spore wurden in einer Einwirkzeit von 150 Minuten abgetötet.

Vergleichbare Resultate wurden auch mit Peressigsäure-Lösungen bestehend aus 4 % Peressigsäure und 96 % Wasser und aus 7 % Peressigsäure und 93 % Wasser erzielt. Bei reinem Aethylengas konnten hingegen unsterile Proben festgestellt werden (z. B. Pseudomonas aerg.).

Weil bei Sterilisierung in einer Formaldehyd-Umgebung festgestellt werden konnte, dass Hitzeaktivierung von bakteriellen Sporen möglich ist, wurde auch dies überprüft. Die benützte Aktivierungstemperatur war 70 °C und die Einwirkungszeit betrug 2 Stunden. Auch nach 2-tägiger Bebrütung der Keime bei 37 °C konnte keine Vermehrung festgestellt werden.

Damit konnte bewiesen werden, dass die gestellte Aufgabe nach einem sicheren, schonenden und ökonomischen Verfahren gelöst werden konnte.

Indem die Entfernung des Aethanol-Anteils von 33 % eine wesentliche Absenkung der Explosibilität des Dampfgemisches erbrachte, die Erhöhung der Peressigsäurekonzentration bis auf 7 % hingegen keine feststellbaren Aenderungen der mikrobiologischen Resultate zeitigte, kann ausgesagt werden, dass die für Menschen unschädliche Konzentration von 4 % Peressigsäure in 96 % Wasser sichere Resultate liefert.

**Patentansprüche**

1. Verfahren zum Sterilisieren von Utensilien, insbesondere von Utensilien aus thermolabilem Material oder mit Bestandteilen aus solchen Materialien, dadurch gekennzeichnet,

dass das zu sterilisierende Gut zuerst in Sterilbeutel verpackt und dann in eine luftdicht abschliessbare Kammer eingebracht wird,

dass darauf die Luft in der Kammer bis auf einen Druck im Bereich von 10-100 mbar evakuiert wird

und wenigstens das Gut auf 40-42 Grad Celsius erwärmt wird,

dass dann Peressigsäurelösung in einer Konzentration im Bereich von 4-7 % Peressigsäure und 93-96 % Wasser in der Kammer bei der genannten Temperatur zur Verdampfung gebracht wird, so dass bei einer Konzentration der verdampften Peressigsäure zwischen 0,1 bis 3,0 mg/Liter ein selbständiger Druckanstieg auf 50-140 mbar bei 40 °C bewirkt wird, und

dass das verpackte Gut während einer Sterilisierzeit von höchstens 150 Minuten in dieser Umgebung belassen wird.

2. Verfahren nach Patentanspruch 1, dadurch gekennzeichnet, dass nach der Sterilisierzeit Reste der Peressigsäure abgesaugt werden.

3. Verfahren nach Patentanspruch 2, dadurch gekennzeichnet, dass vor der Entnahme des Sterilisiergutes die Kammer mit Luft durchspült wird.

### Claims

1. A process for sterilizing utensils, more particularly utensils made of a thermolabile material or having parts made of such materials, characterised in that

the material for sterilization is first packed in sterile bags, then introduced into a chamber adapted to be closed in airtight manner,

whereafter the air in the chamber is exhausted to a pressure in the range of 10-100 mbar

and at least the material is heated to 40-42 °C,

whereafter peracetic acid solution in a concentration of approximately from 4 to 7 % of peracetic acid and from 93 to 96 % of water is introduced into the chamber at the latter temperature for evaporation so that at a concentration of the evaporated peracetic acid of from 0.1 to 3.0 mg/litre an independent pressure increase to 50-140 mbar at 40 °C is produced, and

the packed material is left in this environment for a sterilizing period of at most 150 minutes.

2. A process according to claim 1, characterised in that peracetic acid residues are removed by suction after the sterilizing period.

3. A process according to claim 2, characterised in that the chamber is scavenged with air before removal of the sterilized material.

### Revendications

1. Procédé de stérilisation d'ustensiles, notamment d'ustensiles en matériaux thermolabiles ou ayant des constituants en matériaux de ce type, caractérisé en ce qu'il consiste

à emballer d'abord le produit à stériliser dans un sachet stérile et à le mettre ensuite dans une chambre pouvant être fermée d'une manière étanche à l'air,

à évacuer ensuite l'air de la chambre jusqu'à une pression de l'ordre de 10 à 100 mbar, et à porter le produit au moins entre 40 et 42° Celsius,

à faire ensuite s'évaporer une solution d'acide peracétique en une concentration de l'ordre de 4 à 7 % d'acide peracétique et de 93 à 96 % d'eau dans la chambre à la température mentionnée, de manière à provoquer, pour une concentration de l'acide acétique évaporé comprise entre 0,1 et 3,0 mg/litre, une élévation indépendante de pression jusqu'à 50 à 140 mbar à 40 °C, et

à laisser le produit emballé dans cet environnement pendant une durée de stérilisation de 150 minutes au plus.

2. Procédé suivant la revendication 1, caractérisé en ce qu'il consiste à aspirer les restes de l'acide peracétique après la durée de stérilisation.

3. Procédé suivant la revendication 2, caractérisé en ce qu'il consiste à balayer la chambre par de l'air avant d'enlever le produit stérilisé.

# Fig. 1

## Fig. 2

1. Türe verriegeln
2. Evakuation
3. PES- Injektion
4. Sterilisation
5. Druckausgleich
6. Absaugen
7. Türe entriegeln
8. Belüftung

0    50    100    150   t (min)

## Fig. 3

p (mbar)
1000
500
40
0

$T = 40°C$

0    50    100    150   t (min)